# EUROPÄISCHE PATENTSCHRIFT

(11) **EP 2 613 812 B1**
(45) Veröffentlichungstag und Bekanntmachung des Hinweises auf die Patenterteilung: **14.02.2018**
(21) Anmeldenummer: 11751543.7
(22) Anmeldetag: 09.08.2011
(51) Int. Cl.: A61L 2/02, B01D 71/34, B01D 71/68, B01D 67/00

(54) **STRAHLUNGSRESISTENTE MIKROPORÖSE MEMBRAN MIT HYDROPHOBIZITÄTSGRADIENT**
RADIATION-RESISTANT MICROPOROUS MEMBRANE HAVING A HYDROPHOBICITY GRADIENT
MEMBRANE MICROPOREUSE RÉSISTANT AU RAYONNEMENT, PRÉSENTANT UN GRADIENT D'HYDROPHOBICITÉ

(30) Priorität: 07.09.2010 DE 102010044648
(43) Veröffentlichungstag der Anmeldung: 17.07.2013
(73) Patentinhaber: Sartorius Stedim Biotech GmbH, 37079 Göttingen (DE)
(72) Erfinder: WÜNN, Eberhard, 37077 Göttingen (DE); SCHLEUSS, Tobias, 37085 Göttingen (DE)
(86) Internationale Anmeldenummer: PCT/EP2011/003971
(87) Internationale Veröffentlichungsnummer: WO 2012/031653

(56) Entgegenhaltungen:
- WO-A1-2005/048388
- WO-A1-2009/065092
- WO-A1-2009/086347
- US-A- 5 554 414

## Beschreibung

Die vorliegende Erfindung betrifft eine strahlungsresistente mikroporöse Membran mit einem Hydrophobizitätsgradienten, ein Verfahren zur Herstellung derselben, die Verwendung der Membran in der Sterilfiltration von gasförmigen Fluiden und die Verwendung der Membran als Flüssigkeitssperre in zu belüftenden, flüssigkeitshaltigen Systemen.

In den Bereichen der Nahrungsmittel- und pharmazeutischen Industrie sowie der biotechnologischen Produktion und im Gesundheitswesen ist steriles Arbeiten Voraussetzung für den wirtschaftlichen, Erfolg. Wichtig für das keimfreie Arbeiten ist die Sterilität sowohl von zu prozessierenden Lösungen wie auch die Keimfreiheit von Arbeits- und Betriebsmitteln. Zur letzteren Gruppe zählen aus Metall, Glas oder Kunststoff gefertigte Ansatz-, Misch-, Transport- oder Lagerbehälter, sowie Reaktoren und Fermenter, insbesondere solche mit flexiblen Kunststoffwänden zum Einmalgebrauch.

Gebräuchliche Verfahrensschritte im industriellen Betrieb von metallenen wiederverwendbaren Containern sind Reinigung und Sterilisieren mit Heißdampf, Befüllen, Temperieren, Transport und Entleeren von Flüssigkeiten. Mit Ausnahme des Reinigungsschrittes erfordern die angeführten Prozesse an mindestens einer Behälteröffnung (einem Flansch) ein steril filtrierendes Belüftungselement ("venting device"), um Überdruck- bzw. Vakuumschäden am Betriebsmittel zu verhindern und gleichzeitig die Keimfreiheit des lösungsberührten Innenraumes bei Belüftung zu gewährleisten.

Das Belüftungselement ist die Schnittstelle zwischen einem vorzugsweise sterilen, flüssigkeitshaltigen Behälterinnenraum (z. B. als Flüssigkeitssperre in Dialysevorrichtungen, Infusionslösungsbehältern oder in Fermentern) und äußerer, vorzugsweise unsteriler Atmosphäre. Als eigentliches Trennmedium im Belüftungselement wird in den meisten Fällen ein steril filtrierender Membranfilter aus einem synthetischen Polymer gewählt. In seltenen Fällen wird ein Vliesstoff aus synthetischem Fasermaterial eingebaut.

Synthetische Polymere haben in den meisten Fällen hydrophobe Oberflächeneigenschaften, die auf die intrinsische Hydrophobizität der synthetischen Materialien zurückzuführen ist. Die Hydrophobizität ist eine Materialkonstante. Sie wird durch die extramolekularen Wechselwirkungen der das Polymer aufbauenden Atomgruppen hervorgerufen.

Oberflächen mit einem Kontaktwinkel von mehr als 90° gegenüber Wasser werden als hydrophob bezeichnet. Hydrophobe Substanzen sind nicht mit Wasser mischbar bzw. benetzbar. Die Substanzen sind meist unpolar. Ihre Oberflächenspannung liegt bei 20 °C unter 72 mN/m.

Der hydrophobe Charakter des steril filtrierenden Trennmediums ist aus zweierlei Gründen Voraussetzung für den Einbau in ein Belüftungselement. Zum einen darf sich bei Kontakt mit Wasser bzw. Medium oder insbesondere Wasserdampf (bei Bedampfung oder Begasung von Bioreaktoren) kein geschlossener Wasserfilm auf der Oberfläche bzw. innerhalb des Trennmediums bilden. Der Wasserfilm würde den Druckaustausch (Gasaustausch) zwischen innerer und äußerer Atmosphäre verhindern und dadurch die mechanische Integrität des Behälters gefährden. Eine starke Hydrophobizität (z. B. wie bei fluorhaltigen organischen Polymeren) bis hin zum oleophoben Charakter des Trennmediums ist in diesem Fall vorteilhaft.

So wird bei Belüftungsanwendungen auf gebräuchliche Materialien für Membranfilter, wie Polytetrafluorethylen (PTFE), Polypropylen (PP) und Polyvinylidenfluorid (PVDF), bei Fasermaterial auf Polyethylen (PE) zurückgegriffen.

Zum anderen erlaubt ein hydrophobes Trennmedium die Benetzung mit einfachen organischen Lösungsmitteln (z. B. Alkohol), um die Integrität des Trennmediums vor und nach der Nutzung zu überprüfen. Die Überprüfung des Trennmediums erfolgt dabei anhand des Druckhaltetests und/oder des "Bubble-Point-Tests" (auch Blasendruck-Test). Zum Druckhaltetest wird die benetzte Membran mit Druck beaufschlagt. Man wählt dazu beispielsweise bis etwa 80 % des zu erwartenden "Bubble-Point"-Werts. Dieser Druck wird anschließend einige Minuten beobachtet. In dieser Zeit darf der Druckabfall eine bestimmte Grenze nicht überschreiten. Danach kann unter fortgesetzter Erhöhung des Drucks der "Bubble-Point" bestimmt werden. Im selben Moment, in dem ein kontinuierlicher Luftblasenaustritt auf der drucklosen Seite der Membran zu erkennen ist, wird der erreichte Druck am Manometer abgelesen. Unter Berücksichtigung der Membraneigenschaften können anschließend die größte Pore berechnet und die Rückhalteeigenschaft der Membran abgeschätzt werden.

In vorgenanntem Fall ist ein durchweg oleophobes Filtermedium nachteilig, weil die einfache homogene Benetzung mit vielen für die Integritätstests gebräuchlichen organischen Lösungsmitteln nicht möglich ist.

Die Integrität (= fehlerfreie Versiegelung des Filtermediums im Filtrationsgehäuse, größte Pore zur Abschätzung der Rückhalteeigenschaften) von Filtrationsprodukten mit oleophobem Filtermedium lässt sich durch Bestimmung des Intrusionsdruckes ermitteln. Dabei nutzt man die Eigenschaft von druckbeaufschlagten Flüssigkeiten, deren Oberflächenspannung größer ist als die des nicht benetzenden porösen Systems, bei Erreichen eines Mindestdruckes (= Intrusionsdruck) in die Poren ein- und diese konvektiv zu durchdringen. Der Intrusionsdruck ist um so höher, je kleiner der Radius der zuerst durchdrungenen größten Pore ist. Dazu wird die unter Normalbedingungen (Raumtemperatur, Atmosphärendruck) nicht benetzende Oberfläche des Filtermediums mit der Prüfflüssigkeit vollständig überschichtet. Analog der "Bubble-Point"-Methode zur Ermittlung des Blasendurchtrittspunktes an benetzenden Systemen wird die Flüssigkeit mit zunehmendem Druck beaufschlagt. Sobald Prüfflüssigkeit auf der drucklosen Seite des Filtermediums erscheint, ist der Intrusionsdruck erreicht, dessen Höhe ein Maß für den Radius (Durchmesser) der größten Pore im oleophoben Filtermedium ist.

Mit demselben experimentellen Aufbau lässt sich ein Druckhaltetest durchführen, bei dem die Prüfflüssigkeit mit einem Druck von ca. 80 % des zu erwartenden Intrusionsdruckes beaufschlagt wird.

Oleophobe Substanzen, die sich durch eine besonders hohe Hydrophobizität auszeichnen, sind nicht mit Ölen und anderen unpolaren Stoffen mischbar oder benetzbar. Ihre Oberflächenspannung ist bei 20 °C kleiner als 21 mN/m.

Nur in wenigen Anwendungen werden Wasser oder rein wässrige Lösungen von Salzen (z. B. 0.9 % NaCl, Puffer) prozessiert. Vielfach sind es auf Wasser basierende Rezepturen, die neben anorganischen Salzen auch Netzmittel, organische Lösungsmittel, Proteine, Vitamine und Nährstoffe enthalten, die in der Gesamtheit die Oberflächenspannung des verwendeten Lösungsmittels senken und damit ihr Benetzungsverhalten gegenüber Feststoffen verändern. In diesen Fällen ist es empfohlen, das poröse Trennmedium probehalber mit der Flüssigkeit in Kontakt zu bringen, um das Benetzungsverhalten zu prüfen.

Im Stand der Technik sind verschiedene Verfahren zur Bereitstellung von Membranen mit sowohl hydrophoben als auch oleophoben Eigenschaften beschrieben.

US 2008/0237117 A1 beschreibt asymmetrische Membranen mit einer mehrschichtigen Struktur, die aus einer hydrophoben Basismembran bestehen. Die hydrophobe Basismembran kann aus beliebigen hydrophoben Polymeren, z. B. aus expandiertem PTFE, gebildet sein.
Die hydrophobe Membran weist auf ihrer einen Hauptoberfläche eine diskontinuierliche, die Poren nicht verschließende Beschichtung aus einem oleophoben Polymer (z. B. ein fluoriertes Polymer) auf.
Die derartig einseitig oleophobisierte Membran kann entweder auf ihrer zweiten gegenüberliegenden hydrophoben Hauptoberfläche eine kontinuierliche, die Poren bedeckende, hydrophile Beschichtung aufweisen oder sie ist über einen Klebstoff auf eine zweite hydrophobe Membran mit gleichfalls oleophober Beschichtung so laminiert, daß die beiden oleophoben Beschichtungen des Membrankomposits nach außen weisen. US 2008/0237117 A1 beschreibt weder Membranen mit graduell abgestuften hydrophoben Eigenschaften über den Membranquerschnitt hinweg noch Herstellungsverfahren für Membranen mit einem Hydrophobizitätsgradienten.

WO 2009/065092 A1 offenbart mikroporöse textilverstärkte Polyolefinmembranen aus PE, deren Hauptoberflächen durch ein Imprägnierverfahren selektiv hydrophob bzw. oleophob ausgestattet sind.

Durch das genannte Imprägnierverfahren ist es möglich, eine Hauptoberfläche der mikroporösen PE-Membran mit einem Fluorsubstituenten enthaltenden Polymer oleophob zu machen, während die gegenüberliegende Hauptoberfläche der PE-Membran ihre hydrophoben Ausgangseigenschaften behält.

Nachteilig bei diesen aus WO 2009/065092 A1 bekannten Membranen, die sich als atmungsaktive Materialien bei der Bekleidungsherstellung prinzipiell bewährt haben, ist, daß sie keine ausreichende Beständigkeit gegenüber energiereicher Strahlung, z. B. Gamma-Strahlung, und eine nur unzureichende Temperaturstabilität aufweisen.

US 5,554,414 offenbart mikroporöse Kompositmembranen, deren gesamte innere und äußere Oberfläche mit einem vernetzten Polymer beschichtet ist, welches aus einem Fluorsubstituenten enthaltenden Monomeren und einem Vernetzer gebildet ist.
Die Beschichtung der mikroporösen Membran bewirkt, daß diese mit einer Flüssigkeit mit einer Oberflächenspannung von größer als 21 mN/m nicht benetzbar ist. US 5,554,414 beschreibt weder Membranen mit graduell abgestuften hydrophoben Eigenschaften über den Merribranquerschnitt hinweg (d. h. mit einem Hydrophobizitätsgradienten) noch Herstellungsverfahren für diese.

US 6,579,342 B2 offenbart Belüftungsfilter, deren hydrophobes Basismaterial (z. B. Polysulfon oder PVDF) mit einem oleophoben Oligomer, welches mit Perfluoralkylgruppen und Sulfongruppen funktionalisiert ist, beschichtet ist. Die Beschichtung wird durch Pfropfen des vorgenannten Oligomers auf die Oberfläche des Basismaterials aufgebracht. Auch dieses Dokument offenbart keine Membranen mit Hydrophobizitätsgradient.

WO 2009/086347 A1 offenbart funktionalisierte Membranen, deren innere und äußere Oberflächen durch elektronenstrahlinduzierte Pfropfung mindestens eines Monomertyps auf (Meth)Acrylat-Basis modifiziert sind, und ein Verfahren zu deren Herstellung, wobei die Membranen einen Gradienten von auf die Membranen gepfropften Molekülen aufweisen. Durch gezielte Verschiebung des Maximums des Dosis-Tiefen-Profils der Elektronenstrahlung entlang des Membranquerschnitts und in Abhängigkeit zum Abstand zur Elektronenstrahlquelle lassen sich entlang des Membranquerschnitts verschiedene Gradienten für die Konzentration des Pfropfpolymers einstellen.

In den letzten Jahren hat sich der Trend zur "Single-use"-Nutzung von Kunststoffbehältnissen bei der Prozessierung von Flüssigkeiten verstärkt. Im Unterschied zu den Metallbehältern werden Behälter aus organischen Polymeren zum Zweck der Sterilisierung nicht autoklaviert, sondern üblicherweise durch energiereiche Strahlung, z. B. durch Gamma-Strahlung, keimfrei für die Verwendung vorbereitet. Die Bestrahlung ist ein physikalischer Vorgang, der bei Raumtemperatur stattfindet. Die sterilisierende (abtötende) Wirkung der energiereichen Strahlung beruht auf Bindungsspaltung innerhalb der von Gamma-Strahlung durchdrungenen organischen Materie.

Organische Polymere werden durch energiereiche Strahlung unterschiedlich geschädigt. Insbesondere Polytetrafluorethylen (PTFE), Polypropylen (PP) und Polyvinylchlorid (PVC) erfahren eine dramatische Schwächung ihrer mechanischen Stabilität, wogegen aromatische Polymere wie Poly(ether)sulfone (Polysulfon (PSU), Polyethersulfon (PES)) und Polyimide (PI) nur geringe Veränderungen zeigen. Eine mittlere Toleranz für Gamma-Strahlung zeigen z. B. Polyethylen (PE), Polyester (PET) und Polyvinylidenfluorid (PVDF) (vgl. Tab 1).

**Tab. 1**

| Organisches Polymer | max. kurzzeitige Gebrauchstemperatur (°C)** | Beständigkeitsbereich (kGy)* |
|---|---|---|
| PTFE | 300 | 5 |
| POM | 150 | 15 |
| PP | 140 | 20 |
| PVC | 100 | 50 |
| PA 6.6 | 200 | 50 |
| PMMA | 100 | 100 |
| PE | 120 | 500 |
| PVDF | 150 | 1000 |
| PS | 90 | 1000 |
| PC | 160 | 1000 |
| PET | 200 | 1000 |
| PBT | 165 | 1000 |
| PEEK | 300 | 10000 |
| PI | 400 | 10000 |
| PAI | 300 | 10000 |
| PSU | 170 | 10000 |
| PES | 260 | 10000 |

| | | |
|---|---|---|
| POM: Polyoxymethylen; PA 6.6: Polyamid 6.6, Polyhexamethylenadipamid; PMMA: Polymethylmethacrylat; PC: Polycarbonat, PAI: Polyamidimid, PEEK: Polyetheretherketon, PS: Polystyrol, PBT: Polybutylenterephthalat * Beständigkeitsbereich bei Gamma-Bestrahlung: Datenblatt des BGS (Bezugsquelle: Beta-Gamma-Service GmbH & Co. KG, Fritz-Kotz-Straße 16, D-51674 Wiehl) ** Temperaturangaben aus "Saechtling Kunststoff Taschenbuch", Hrsg. K. Oberbach, C. Hanser Verlag, 27. Ausgabe, Tab. 5.14 | | |

Eine Zusammenfassung der Materialeigenschaften von heute gebräuchlichen porösen Trennmedien aus Tab. 1 offenbart, dass keines der Materialien der Summe der Anforderungen genügen kann und somit als universelles Filtermedium für sterile Belüftungsanwendungen genutzt werden kann: Das hydrophobe PTFE zeigt bei ausgezeichneter Temperaturstabilität die geringste Belastbarkeit durch sterilisierende Strahlungsbehandlung. Das hydrophobe PE ist wegen geringer Temperaturstabilität und das hydrophobe PP wegen unzureichender Strahlungsbelastbarkeit benachteiligt (vgl. Tab. 1).

Energiereiche Strahlung führt bei Polymeren mit rein aliphatischer Hauptkette (z. B. PE, PTFE) oder bei solchen mit mindestens zwei aufeinanderfolgenden gesättigten Kohlenstoffatomen zwischen aromatischen Kettensegmenten (z. B. PET, PC) zu stärkerer Beeinträchtigung ihrer mechanischen Festigkeit als bei aromatischen Hauptkettenpolymeren, die ausschließlich aus aromatischen Bausteinen, aus einer von nur einem nichtaromatischen Hauptkettenatom unterbrochenen Aromatenkette (z. B. PSU, PES) oder aus aromatischen Bausteinen aufgebaut sind, welche durch ein nicht aromatisches Ringsystem miteinander verbunden sind.

Die Mehrzahl der in der Literatur für polymere Werkstoffe beschriebenen Beständigkeitsbereiche für Gamma-Strahlung basiert auf mechanischen Messungen an massiven Formkörpern (z. B. Datenblatt des BGS (Beta-Gamma-Service, D-51674 Wiehl)). Filtermedien sind poröse Folien oder faserige Materialien, deren innere Porosität zwischen 50 % und 80 % beträgt. Die geringe Materialdichte der porenbildenden Matrix erhöht den Festigkeitsverlust poröser Trennmedien durch die schädigende Wirkung der energiereichen Strahlung.

Der vorliegenden Erfindung liegt die Aufgabe zugrunde, eine mikroporöse Membran bereitzustellen, deren erste äußere oleophobe Hauptoberfläche mit hydrophilen Substanzen, wie niederen Alkoholen (z. B. Ethanol, Isopropanol) oder Detergenzien enthaltenden wäßrigen Lösungen, nicht benetzbar ist, während die zweite äußere hydrophobe Hauptoberfläche der Membran mit den vorgenannten hydrophilen Substanzen benetzbar ist und einem Integritätstest bzw. einer Prüfbarkeit zugänglich ist, wobei die erfindungsgemäße Membran gleichzeitig eine Temperatur- und Strahlungsbehandlung zulässt.

Diese Aufgabe wird durch die Bereitstellung einer strahlungsresistenten mikroporösen Membran mit einem Hydrophobizitätsgradienten nach Anspruch 1, der Verwendung der Membran in der Sterilfiltration von gasförmigen Fluiden nach Anspruch 9 und der Verwendung der Membran als Flüssigkeitssperre in zu belüftenden flüssigkeitshaltigen Systemen nach Anspruch 10 gelöst.

Unter mikroporösen Membranen im Sinne der vorliegenden Erfindung sollen Membranen mit einer Porengröße zwischen 0,01 bis 10 µm verstanden werden.

Die mikroporöse Membran gemäß der vorliegenden Erfindung ist eine mikroporöse, gegenüber Gammastrahlung resistente, temperaturbeständige Polymermembran mit zwei äußeren Hauptoberflächen, die durch den Membrankörper über Mikroporen verbunden sind, und mit einer maximalen kurzzeitigen Gebrauchstemperatur von mindestens 150 °C, wobei die mikroporöse Membran von der ersten äußeren Hauptoberfläche durch den Membrankörper zu der zweiten äußeren Hauptoberfläche einen Hydrophobizitätsgradienten aufweist, wobei der Hydrophobizitätsgradient dergestalt ist, dass die Hydrophobizität auf der ersten äußeren Hauptoberfläche maximal ist, zur zweiten äußeren Hauptoberfläche hin zunächst abnimmt, innerhalb des mikroporösen Membrankörpers ein Minimum annimmt, jedoch noch vorhanden ist, und anschließend zur zweiten äußeren Hauptoberfläche hin wieder zunimmt, ohne das Maximum der ersten äußeren Hauptoberfläche wieder zu erreichen.

Bei einer bevorzugten Ausführungsform der erfindungsgemäßen Membran ist die erste äußere Hauptoberfläche oleophob und die zweite äußere Hauptoberfläche hydrophob.
Durch diese Eigenschaftskombination ist die erfindungsgemäße Membran in besonderer Weise für die Sterilfiltration von gasförmigen Fluiden oder als Flüssigkeitssperre für zu belüftende Systeme geeignet, indem sie oberseitig oleophob weder von tensidhaltigen Lösungen noch von niederen Alkoholen (z. B. Ethanol, Isopropanol) benetzt werden kann, von der hydrophoben Unterseite hingegen zur Überprüfung der Integrität ("Bubble-Point"-Test, Druckhaltetest) mit den vorgenannten Alkoholen oder tensidhaltigen Lösungen benetzt werden kann.

Die erfindungsgemäße Membran zeigt eine maximale kurzzeitige Gebrauchstemperatur von mindestens 150 °C und am meisten bevorzugt von mindestens 180 °C. Unter "maximaler kurzzeitiger Gebrauchstemperatur" wird gemäß "Saechtling Kunststoff Taschenbuch", Hrsg. K. Oberbach, C. Hanser Verlag, 27. Ausgabe, und gemäß der vorliegenden Erfindung die Höchsttemperatur verstanden, bis auf welche die erfindungsgemäße Membran vorübergehend erhitzt werden kann, ohne daß sie beim Wiederabkühlen auf Raumtemperatur eine Veränderung ihrer mechanischen Eigenschaften zeigt, d. h. ihre mechanischen Eigenschaften nach dem Erhitzen auf die maximale kurzzeitige Gebrauchstemperatur und nach dem Wiederabkühlen auf Raumtemperatur entsprechen den mechanischen Eigenschaften einer erfindungsgemäßen Membran, welche nicht vorübergehend auf diese Gebrauchstemperatur erhitzt worden ist. Beim Überschreiten der maximalen kurzzeitigen Gebrauchstemperatur treten dahingegen Veränderungen der mechanischen Eigenschaften auf.

Im Speziellen beschreibt die vorliegende Erfindung eine mikroporöse Membran, beispielsweise für die Sterilfiltration von Gasen oder auch als Flüssigkeitssperre für zu belüftende Systeme, deren eine Hauptoberfläche oleophobe Eigenschaften aufweist, während ihre zweite Hauptoberfläche hydrophobe Eigenschaften aufweist, und die eine maximale kurzzeitige Gebrauchstemperatur (vgl. "Saechtling Kunststoff Taschenbuch", Hrsg. K. Oberbach, C. Hanser Verlag, 27. Ausgabe) von größer als 150 °C zeigt und durch sterilisierende Gamma-Strahlung in Dosen bis einschließlich 50 kGy keine schädliche Verringerung der mechanischen Eigenschaften erfährt.

Die erfindungsgemäße Membran ist sowohl durch 20minütige Behandlung mit Heißdampf bei 134 °C als auch durch Bestrahlung mit keimabtötender Gammastrahlung in einer Dosis von 50 kGy sterilisierbar. Während vor Jahren z. B. die Sterilisation mittels Heißdampfbehandlung im Autoklaven bei 121 °C (entspricht einem Überdruck von 0,1 MPa) akzeptiert war, wird heute eine Temperatur von 125 °C bis 134 °C (entspricht einem Überdruck von 0,2 MPa) angewandt, um Mikroorganismen (insbesondere in Produktionsanlagen) mit ausreichender Sicherheit abzutöten. Dieser Wandel im Sicherheitsverständnis führt dazu, dass Werkstoffe mit einer kurzzeitigen maximalen Gebrauchstemperatur kleiner 125 °C ("Saechtling Kunststoff Taschenbuch", Hrsg. K. Oberbach, C. Hanser Verlag, 27. Ausgabe, z. B. PVC, PS, PE, Tab. 5.14) nicht mehr verwendet werden.

Gemäß einer bevorzugten Ausführungsform der erfindungsgemäßen Membran beträgt der Festigkeitsverlust der Membran nach Gamma-Bestrahlung mit einer Dosis von 50 kGy nicht mehr als 30 %, bevorzugt nicht mehr als 20 % und besonders bevorzugt nicht mehr als 10 %. Der Festigkeitsverlust der erfindungsgemäßen Membran ergibt sich dabei aus der Abnahme des Festigkeitswerts der Membran nach der Gamma-Bestrahlung mit einer Dosis von 50 kGy bezogen auf den Festigkeitswert der Membran vor der Gamma-Bestrahlung mit dieser Dosis. Beträgt der Festigkeitswert der bestrahlten Membran 80 % des Festigkeitswertes der unbestrahlten Membran, beträgt der Festigkeitsverlust erfindungsgemäß 20 %. Die Festigkeitswerte der bestrahlten und unbestrahlten Membranen werden im Sinne der vorliegenden Erfindung durch ihre maximalen Zugkraftwerte Fₘₐₓ bei Raumtemperatur beschrieben (vgl. Tabelle 2A und 2B).

Die erfindungsgemäße Membran toleriert einfache und mehrfache Sterilisation durch Gammastrahlung. Die Gammabestrahlung erfolgt bevorzugt in Sammelgebinden, die zum Erreichen einer Mindestdosis von 50 kGy während 4 bis 8 Stunden um eine Strahlenquelle herum bewegt werden.

Der Hydrophobizitätsgradient ist erfindungsgemäß dergestalt, dass die Hydrophobizität auf der ersten äußeren Hauptoberfläche der mikroporösen Membran maximal ist, zur zweiten äußeren Hauptoberfläche hin zunächst abnimmt, innerhalb des mikroporösen Membrankörpers ein Minimum annimmt, jedoch noch vorhanden ist, und anschließend zur zweiten äußeren Hauptoberfläche hin wieder zunimmt, ohne das Maximum der ersten äußeren Hauptoberfläche wieder zu erreichen.

Bei einer bevorzugten Ausführungsform der erfindungsgemäßen mikroporösen Membran beträgt die Oberflächenspannung der Flüssigkeit, durch welche die erste äußere Hauptoberfläche benetzbar ist, weniger als 21 mN/m, während die Oberflächenspannung der Flüssigkeit, durch welche die zweite äußere Hauptoberfläche benetzbar ist, zwischen 23 und 40 mN/m liegt.

Der gemäß der vorliegenden Erfindung in der mikroporösen Membran vorliegende Hydrophobizitätsgradient ist bevorzugt durch die Anwesenheit einer fluorhaltigen organischen Verbindung erzeugt. Der durch die Anwesenheit der fluorhaltigen organischen Verbindung verursachte Gradient im Fluorgehalt innerhalb des Membrankörpers als Indikator für den Hydrophobizitätsgradienten kann dabei in der erfindungsgemäßen mikroporösen Membran durch EDX-Analyse ("Energydispersive X-ray Spectroscopy", d.h. energiedispersive Röntgenspektroskopie) ermittelt werden.

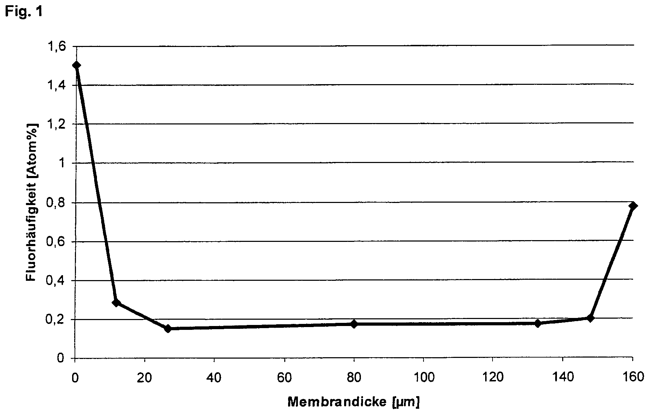

Die EDX-Analytik nutzt die charakteristische hochenergetische elektromagnetische Strahlung (= Röntgenstrahlung), die Elemente bei Elektronenbeschuss abgeben. Die Strahlung wird freigesetzt, wenn ein kernnahes Elektron nach Kollision mit einem Fremdelektron in die kernnahe K- oder L-Schale zurückfällt. Die dabei freiwerdende Energie in Form hochfrequenter elektromagnetischer Schwingung ist spezifisch für das zu analysierende Element (hier Fluor) und kann von externen Detektoren analysiert werden. Die EDX-Analytikmethode ermöglicht es, den Hydrophobizitätsgradienten der erfindungsgemäßen Membran, welcher von der ersten äußeren Hauptoberfläche zu der zweiten äußeren Hauptoberfläche durch den Membrankörper verläuft, als Atomverteilung in Atom% der Fluorsubstituenten der fluorhaltigen organischen Verbindung nachzuweisen (vgl. Figur 1).

Die vorstehend beschriebene fluorhaltige organische Verbindung liegt dabei bevorzugt in Form eines an der mikroporösen Membran fixierten, Fluorsubstituenten enthaltenden Polymers vor.

Bei einer alternativen Ausführungsform der erfindungsgemäßen Membran ist die fluorhaltige organische Verbindung ein Reaktionsprodukt einer monomeren, oligomeren oder polymeren fluorhaltigen Verbindung mit reaktiven Gruppen des Ausgangsmaterials der Polymermembran.
Die Eindringtiefe (Porengröße des Ausgangsmaterials der Polymermembran vs. Teilchengröße der fluorhaltigen Verbindung) der zugrunde liegenden monomeren, oligomeren oder polymeren fluorhaltigen Verbindung in das Ausgangsmaterial der Polymermembran bestimmt dabei die Fluorverteilung und damit den Hydrophobizitätsgradienten in der erfindungsgemäßen mikroporösen Membran.

Besonders bevorzugt sind Ausführungsformen, bei welchen die erste äußere Hauptoberfläche der erfindungsgemäßen Membran oleophob ist und die erste Hauptoberfläche die fluorhaltige Verbindung in höherer Konzentration enthält als die zweite äußere, hydrophobe Hauptoberfläche und als der mikroporöse Membrankörpers, so daß die erste äußere Hauptoberfläche nur mit Flüssigkeiten mit einer Oberflächenspannung kleiner als 21 mN/m benetzbar ist, während die zweite äußere Hauptoberfläche sowie die Oberfläche innerhalb des Membrankörpers durch Flüssigkeiten mit einer Oberflächenspannung von höchstens 40 mN/m benetzbar sind.

Bei einer bevorzugten Ausführungsform der Erfindung besteht das Ausgangsmaterial der Polymermembran aus PVDF, PSU (Polysulfon), PES (Polyethersulfon), PPS (Polyphenylensulfid), PBI (Polybenzimidazol), PEEK oder PAI.

Bei einer besonders bevorzugten Ausführungsform der Erfindung umfaßt die Polymermembran mindestens ein aromatisches Hauptkettenpolymer. Am meisten bevorzugt ist das aromatische Hauptkettenpolymer ausgewählt aus der Gruppe von PSU, PES oder Gemischen hiervon.

Die erfindungsgemäße mikroporöse Membran weist bevorzugt eine Dicke zwischen 50 und 250 µm auf und liegt bevorzugt als Bahnmaterial vor. Die Porengröße beträgt dabei zwischen 0,01 und 10 µm.

Ein Verfahren zur Herstellung einer erfindungsgemäßen mikroporösen Membran umfasst die Schritte des Imprägnierens der mikroporösen Membran durch eine ihrer beiden Hauptoberflächen hindurch mit einer fluorhaltigen organischen Verbindung und des Erwärmens der mikroporösen Membran.

Der erfindungsgemäße Hydrophobizitätsgradient der mikroporösen Membran wird beispielsweise durch Imprägnierung der Membran mit einem reaktiven Textilhilfsmittel und nachfolgende Trocknung und Vernetzung bei Temperaturen im Intervall von 125 °C bis 200 °C herbeigeführt. Reaktive Textilhilfsmittel zur Herbeiführung eines Öl abweisenden Effektes enthalten FluorKohlenstoffverbindungen, die auf die Fasern des Textiles aufgebracht und bei erhöhter Temperatur als Überzug auf der Faseroberfläche verfestigt werden.

Dabei wird beispielsweise die mit dem Textilhilfsmittel imprägnierte flächige mikroporöse Membran getrocknet und nachfolgend auf eine Trocknungstemperatur im Bereich von 120 bis 150 °C, beispielsweise 135 °C, gebracht, bei der die abgelagerten reaktiven Substanzen schmelzen.

Danach wird eine Nachbehandlungstemperatur im Bereich von 150 bis 200 °C, beispielsweise 170 °C, erreicht, bei der die reaktiven Gruppen der geschmolzenen Substanzen aktiviert werden und während ausreichender Verweilzeit bei der Nachbehandlungstemperatur eine Vernetzungsreaktion eingehen.

Danach weisen die äußeren Hauptoberflächen der so behandelten porösen flächigen Membran verschiedene Oberflächenspannungen auf, wobei die Hauptoberfläche mit der höheren Substanzmenge an Textilhilfsmittel eine geringere Oberflächenspannung aufweist als die Hauptoberfläche mit der niedrigeren Substanzmenge an Textilhilfsmittel.

Schließlich stellt die vorliegende Erfindung die Verwendung der erfindungsgemäßen mikroporösen Membran in der Sterilfiltration von gasförmigen Fluiden und die Verwendung der erfindungsgemäßen mikroporösen Membranen als Flüssigkeitssperre in zu belüftenden flüssigkeitshaltigen Systemen bereit.

Die vorliegende Erfindung stellt eine mikroporöse Membran mit einer ersten äußeren, oleophoben Hauptoberfläche und einer zweiten äußeren, hydrophoben Hauptoberfläche bereit, deren erste äußere oleophobe Hauptoberfläche mit hydrophilen Substanzen, wie niederen Alkoholen (z. B. Ethanol, Isopropanol) oder Detergenzien enthaltenden wäßrigen Lösungen, nicht benetzbar ist, während die zweite äußere hydrophobe Hauptoberfläche der Membran mit den vorgenannten hydrophilen Substanzen benetzbar ist und einem Integritätstest bzw. einer Prüfbarkeit zugänglich ist, wobei die erfindungsgemäße Membran gleichzeitig eine Temperatur- und Strahlungsbehandlung zulässt. Damit kann die erfindungsgemäße mikroporöse Membran vorteilhaft in Sterilfiltrationen von gasförmigen Fluiden Anwendung finden, beispielsweise an mindestens einer Behälteröffnung (einem Flansch) als Teil eines steril filtrierenden Belüftungselements ("venting device") oder als Flüssigkeitssperre für flüssigkeitshaltige, zu belüftende Systeme, um Überdruck- bzw. Vakuumschäden am Betriebsmittel zu verhindern und gleichzeitig ggf. bei sterilen Applikationen die Keimfreiheit des lösungsberührten Innenraumes bei Belüftung zu gewährleisten. Dabei wird die mikroporöse Membran einerseits auf ihrer oleophoben, dem Behälterinnenraum zugewandten Seite von der im Inneren befindlichen Lösung und deren Inhaltsstoffen nicht benetzt und bleibt damit gasdurchlässig. Andererseits bietet die hydrophobe Außenseite (d. h. die zweite äußere Hauptoberfläche) der erfindungsgemäßen mikroporösen Membran weiterhin die Möglichkeit, Integritätstests in Form eines Druckhaltetests und/oder "Bubble-Point"-Tests durchzuführen. Gleichzeitig eignet sich eine solche Anordnung, welche die erfindungsgemäße mikroporöse Membran umfasst, vorteilhafterweise zur Sterilisierung mittels Gamma-Bestrahlung für den Einsatz in der Einmalverwendung (z. B. in Einweg-Plastikbeuteln mit sterilen Belüftungsfiltern). Trotz der erfindungsgemäßen Modifikation der mikroporösen Membran mittels eines Hydrophobizitätsgradienten weist die Membran darüber hinaus in der Endverwendung eine ausreichende Luftpermeabilität auf. Die vorliegende Erfindung sowie weitere sich daraus ergebende Vorteile werden unter Bezugnahme auf die in den Beispielen beschriebenen Ausführungsformen näher erläutert, ohne den Schutzbereich des Anspruchsbegehrens auf diese Ausführungsformen einzuschränken.

### Beispiele

### Beispiel 1

Eine hydrophobe 0,2-µm-Mikrofiltrationsmembran aus PVDF (Fa. Millipore, Typ GVHP) wird bei Raumtemperatur mit der ersten äußeren Hauptoberfläche durch Auflegen für 5 Sekunden auf eine verdünnte NUVA 3049 fl (Clariant, 30%ig) Dispersion, bestehend aus 8 Volumenteilen (VT) 30%ig NUVA, 95 VT. Wasser und 20 VT. 2-Propanol, imprägniert. Die benetzte Membranprobe wird dann für weitere 15 Sekunden vollständig in die Dispersion getaucht, anschließend für 1 Minute an der Luft senkrecht gehalten, um überschüssige Imprägnierflüssigkeit ablaufen zu lassen. Die so imprägnierte Mikrofiltrationsmembran wird mit der zweiten äußeren Oberfläche auf eine beheizte Platte abgelegt und bei 125 °C getrocknet und anschließend 10 Minuten bei 150 °C nachbehandelt. Die nach Wärmebehandlung oleophobe erste äußere Oberfläche wird von reinem 2-Propanol nicht benetzt. Die zweite äußere Oberfläche sowie die innere Oberfläche der behandelten Membranprobe zeigen spontane homogene Benetzung mit 2-Propanol.

### Beispiel 2

Die feinporige Seite einer asymmetrischen hydrophoben 0,2-µm-Mikrofiltrationsmembran aus Polysulfon (PSU, Sartorius Stedim Biotech GmbH, Typ 14907) wird durch Auflegen auf eine verdünnte NUVA 3049 fl (Clariant) Dispersion, bestehend aus 6 VT. 30%ig NUVA, 94 VT. Wasser und 20 VT. Ethanol, benetzt, entsprechend Beispiel 1 getrocknet und anschließend bei einer Temperatur von 170 °C nachbehandelt. Die so erhaltene einseitig oleophobe PSU-Mikrofiltrationsmembran benetzt unterseitig (an der unbehandelten, nicht imprägnierten Hauptoberfläche) spontan mit Ethanol, an der behandelten, imprägnierten Oberfläche perlt Ethanol ab.

### Beispiel 3

Eine wasserbenetzbare mikroporöse 0,2-µm-PES-Membran (Sartorius Stedim Biotech GmbH, Typ 15407) wird durch Auflegen auf eine verdünnte NUVA 3049fl Dispersion, bestehend aus 5 TI. 30%ig NUVA und 105 TI. Wasser benetzt und entsprechend Beispiel 1 behandelt, wobei die Nachbehandlung bei 180 °C erfolgt, um anschließend eine einseitig oleophobe Membran zu erhalten.

### Bestimmung des Festigkeitsverlusts bei Gamma-Bestrahlung mit einer Dosis von 50 kGy für erfindungsgemäße Membranen nach Beispiel 2 und 3 bzw. für Vergleichsproben

Zur Bestimmung des Festigkeitsverlusts nach Gamma-Bestrahlung mit einer Dosis von 50 kGy wird ein rechteckiger Membranzuschnitt (1. Membranzuschnitt: Membran ohne Gamma-Bestrahlungsbehandlung, 2. Membranzuschnitt: Membran nach Gamma-Bestrahlung mit 50 kGy) in Längsrichtung in die übereinander (vertikal) angeordneten Aufnahmen (Klemmbacken) einer nachfolgend näher beschriebenen Messapparatur eingespannt. Die obere Klemmbacke ist mit einem Kraftaufnehmer verbunden, der wiederum mit konstanter Geschwindigkeit (Spindelantrieb) vertikal bewegt werden kann. Als charakteristische Beobachtungsgröße wird die maximale Zugkraft Fₘₐₓ für das Dehnen der fest eingespannten Membran herangezogen.

Eine Membranprobe wird hierfür mit den Maßen 20 mm x 150 mm zugeschnitten und horizontal in eine "Zwick Z2.5/TN1S"-Materialprüfmaschine der Fa. Zwick GmbH so eingespannt, dass die freie Probenlänge zwischen den Klemmbacken 4 cm beträgt. Der Kraftaufnehmer "KAP-Z 200N", Fa. A.S.T., D-01287 Dresden, wird mit einer Geschwindigkeit von beispielsweise 5 cm/min bewegt. Die Messdaten werden von der Gerätesoftware "testXpert", Fa. Zwick GmbH, D-89079 Ulm, kontinuierlich erfasst und visualisiert. Fₘₐₓ wird als Mittelwert von drei bestrahlten Membranproben bzw. drei unbestrahlten Membranproben bestimmt (vgl. Tab. 2A und Tab. 2B)

**Tab. 2A**

| Probe | Probennr. |
|---|---|
| PTFE-MF 0,2 µm (Gore Microfiltration Media, US, Typ S30189) | 1) |
| PA-MF 0,2 µm (Sartorius-Stedim Biotech, D, Typ 25007) | 2) |
| PP-MF 0,2 µm (Membrana, D, Typ2 EHF 6037) | 3) |
| PVDF-MF 0,22 µm (Millipore Corp., US, Typ GVHP) | 4) |
| PVDF-MF 0,22 µm gem. Beispiel 1 (Millipore Corp., US, Typ GVHP) | 5) |
| PE Blasfolie | 6) |
| PPS Vliesstoff 120 µm (Carl Freudenberg, D, Typ FO2440-608243) | 7) |
| PET Vliesstoff 110 µm (Hirose, J, Typ 05-TH80) | 8) |
| PET Folie 50 µm (Pütz, D, Typ Hostaphan RN) | 9) |
| PSU-MF 0,2 µm (Sartorius-Stedim Biotech, D, Typ 14907) | 10) |
| PSU-MF 0,2 µm gem. Beispiel 2 (Sartorius-Stedim Biotech, D, Typ 14907 OB) | 11) |
| PES-MF 0,2 µm (Sartorius-Stedim Biotech, D, Typ 15407) | 12) |
| PES-MF 0,2 µm gem. Beispiel 3 (Sartorius-Stedim Biotech, D, Typ 15407 OB) | 13) |

**Tab. 2B**

| | Fₘₐₓ/[N] | | Änderung Fₘₐₓ |
|---|---|---|---|
| Probennr. | unbestrahlt | bestrahlt 50 kGy | % |
| 1) | 10,6 | 1,1 | -90,0 |
| 2) | 17,5 | 9,9 | -43,3 |
| 3) | 4,5 | 2,6 | -41,7 |
| 4) | 12,7 | 10,6 | -16,3 |
| 5) | 12,6 | 10,1 | -19,8 |
| 6) | 25,6 | 24,5 | -4,2 |
| 7) | 86,4 | 83,5 | -3,4 |
| 8) | 98,4 | 105,1 | 6,8 |
| 9) | 130,5 | 126,9 | -2,8 |
| 10) | 8,2 | 8,4 | 2,3 |
| 11) | 9,1 | 9,2 | 1,1 |
| 12) | 12,5 | 12,2 | -2,2 |
| 13) | 15,0 | 15,4 | 2,3 |

Bei den erfindungsgemäßen Membranen gemäß der Beispiele 1, 2 und 3 (Probennummern 5), 11) und 13) beträgt der Festigkeitsverlust nach einer Gammabestrahlung mit einer Dosis von 50 kGy unter 20 % bezogen auf die unbestrahlte Membran, bei den erfindungsgemäßen Membranen auf Polysulfon- bzw. Polyethersulfonbasis wird sogar eine geringfügige Zunahme des Festigkeitswerts um 1,1 % bzw. 2,3 % im Vergleich zur unbestrahlten Membran beobachtet.

### Benetzungsverhalten der erfindungsgemäßen Membranen mit Wasser, wäßrigen Lösungen von NaCl (0,9 Gew%) bzw. eines Detergens (1 Gew%) oder Wasser-Ethanol-Gemischen

Ergebnisse zum Benetzungsverhalten verschiedener mikroporöser (0,2 µm) sterilfiltrierender Membranfilter werden visuell wie nachfolgend beschrieben bestimmt und sind in Tabelle 3 gezeigt. Die Membranprobe wird auf eine von der Rückseite her beleuchtete Milchglasscheibe gelegt. Ein 50-µl-Flüssigkeitstropfen wird mittels Pipette auf die horizontal liegende äußere, oleophobe Hauptoberfläche der Membranprobe aufgetragen. Bei dieser oleophoben Hauptoberfläche handelt es sich bei der erfindungsgemäßen Membran des Beispiels 3 um die Hauptoberfläche, durch welche hindurch die Membran im ersten Schritt des erfindungsgemäßen Verfahrens nach Beispiel 3 mit der fluorhaltigen organischen Verbindung imprägniert worden ist.
"Benetzt": Flüssigkeit ist in die innere Oberfläche eingedrungen. Die Membranprobe erscheint im benetzten Bereich heller. Erfolgt nach 60 Sekunden keine Benetzung der Membranprobe, wird der Flüssigkeitstropfen mittels Pipette aufgesaugt. "Film": Bei Absaugen der Flüssigkeit bleibt ein Film auf der Oberfläche zurück, die Flüssigkeit spreitet auf der Oberfläche und dringt nicht in die innere Oberfläche ein.
"Tropfen (Tr.)": Flüssigkeit bleibt als Tropfen auf der Oberfläche liegen, lässt sich vollständig absaugen. Es erfolgt keine Benetzung der Membran.

**Tab. 3:**

| Material 0,2 µm | Oberflächenspannung σ [mN/m] | H₂O | 0,9 % NaCl | 1 % Detergens | 5 % EtOH | 20 % EtOH | 50 % EtOH | 98 % EtOH |
|---|---|---|---|---|---|---|---|---|
| PES hydrophil* | - | benetzt | benetzt | benetzt | benetzt | benetzt | benetzt | benetzt |
| PSU | - | Tr. | Tr. | Film | Film | Film | benetzt | benetzt |
| PVDF | 33,7 | Tr. | Tr. | Film | Tr. | Film | benetzt | benetzt |
| PP | 32 | Tr. | Tr. | Film | Tr. | Film | benetzt | benetzt |
| PTFE | 22,5 | Tr. | Tr. | | Tr. | Tr. | Film | benetzt |
| PES behandelt** | 21 | Tr. | Tr. | Tr. | Tr. | Tr. | Tr. | Tr. |

| | | | | | | | | |
|---|---|---|---|---|---|---|---|---|
| Alle Prozentangaben für NaCl bzw. EtOH sind Gewichtsprozentangaben für eine wäßrige Kochsalzlösung bzw. für Ethanol in einem Ethanol-Wasser-Gemisch. *Ausgangsmembran des Beispiels 3 ** Erfindungsgemäße Membran hergestellt nach dem erfindungsgemäßen Verfahren des Beispiels 3 mit Hydrophobizitätsgradient | | | | | | | | |

### Nachweis des Hydrophobizitätsgradienten in der erfindungsgemäßen Membran nach Beispiel 3 mit EDX-Analytik

Der Elektronenbeschuss einer nicht leitfähig bedampften, erfindungsgemäßen Membranprobe nach Beispiel 3 erfolgt mit einer Beschleunigungsspannung von 7,5 kV in der Vakuumkammer eines Elektronenmikroskopes der Fa. Fei, Typ "Quanta 200 FEG" im "Low-vacuum mode" bei 0,1 - 1 Torr. Der in die Kammer eingebrachte EDX-Detektor der Fa. EDAX ist ein mit flüssigem Stickstoff gekühlter, mit Lithium dotierter Siliziumkristall. Als Auswertesoftware wird das Programm "Genesis" der Fa. EDAX genutzt.

Zur Analyse des Fluorgehaltes einer Oberfläche werden die zweite äußere, hydrophobe Hauptoberfläche und die zwischen den beiden äußeren Hauptoberflächen aufgespannte innere Oberfläche der erfindungsgemäßen Membran mit einem C₁- bis C₃-Alkanol, z. B. mit 2-Propanol, benetzt und in flüssigem Stickstoff gebrochen. Die Bruchfläche, d. h. die Querschnittsfläche der Membran zwischen den beiden äußeren Hauptoberflächen, wird in der Vakuumkammer eines Rasterelektronenmikroskops (REM) positioniert und so weit vergrößert, dass die äußeren Hauptoberflächen nicht mehr im Suchbereich des abtastenden Elektronenstrahls erscheinen. Der EDX-Detektor nimmt die hochfrequenten Signale der vom Strahl des REM getroffenen Atome auf. Die diskreten Frequenzen und deren Häufigkeit werden von der Software zugeordnet und als Verteilung der Fluoratome über den Membranquerschnitt abgebildet (vgl. Figur 1). Die Angaben in Atom% (EDAX-Software) sind Mittelwerte der Fluorbestimmungen in drei in derselben Höhe nebeneinander liegenden 10x10-µm-Meßfenstern.
Zur Zweipunkt-Kalibrierung der erhaltenen Meßergebnisse werden nach dem beschriebenen Detektionsverfahren nacheinander eine nicht mit einer Fluorverbindung behandelte Ausgangsmembran (z. B. PES; Fluorgehalt 0 %) und eine fluorhaltige Membran (z. B. PTFE, Fluorgehalt 34 %) analysiert.
Die erste äußere Hauptoberfläche der Membran nach Beispiel 3 zeigt den höchsten Fluorgehalt (1,5 Atom%), während innerhalb des Membrankörpers der Fluorgehalt auf einen Minimalwert von unter 0,2 Atom% sinkt, um auf der zweiten äußeren, hydrophoben Hauptoberfläche wieder auf 0,8 Atom% anzusteigen.

Die Ergebnisse der Tabellen 2A, 2B sowie 3 in Verbindung mit Figur 1 belegen, daß die erfindungsgemäßen Membranen aufgrund ihres Hydrophobizitätsgradienten auf ihrer ersten äußeren oleophoben Hauptoberfläche mit hydrophilen Substanzen (wie niedere Alkohole (z. B. Ethanol, Isopropanol), Detergenzien enthaltende wäßrige Lösungen) nicht benetzbar sind, auf ihrer zweiten äußeren hydrophoben Hauptoberfläche aber mit den vorgenannten hydrophilen Substanzen benetzbar sind und einem Integritätstest bzw. einer Prüfbarkeit zugänglich sind. Verbunden mit diesem vorteilhaften Hydrophobizitätsgradienten ist gleichzeitig eine hohe Temperatur- und Gammma-Bestrahlungsbeständigkeit, die die Membran insbesondere für den Einsatz in sterilen Einwegapplikationen der Biotechnologie prädestiniert.

## Patentansprüche

1. Mikroporöse, gegenüber Gammastrahlung resistente, temperaturbeständige Polymermembran mit zwei äußeren Hauptoberflächen, die durch den Membrankörper über Mikroporen verbunden sind, und mit einer maximalen kurzzeitigen Gebrauchstemperatur von mindestens 150 °C, wobei die mikroporöse Membran von der ersten äußeren Hauptoberfläche durch den Membrankörper zu der zweiten äußeren Hauptoberfläche einen Hydrophobizitätsgradienten aufweist, wobei der Hydrophobizitätsgradient dergestalt ist, dass die Hydrophobizität auf der ersten äußeren Hauptoberfläche maximal ist, zur zweiten äußeren Hauptoberfläche hin zunächst abnimmt, innerhalb des mikroporösen Membrankörpers ein Minimum annimmt, jedoch noch vorhanden ist, und anschließend zur zweiten äußeren Hauptoberfläche hin wieder zunimmt, ohne das Maximum der ersten äußeren Hauptoberfläche wieder zu erreichen.

2. Mikroporöse Membran nach Anspruch 1, wobei der Festigkeitsverlust der Polymermembran durch γ-Bestrahlung mit einer Dosis von 50 kGy nicht mehr als 20 % beträgt.

3. Mikroporöse Membran nach einem der Ansprüche 1 oder 2, wobei die erste Hauptoberfläche durch eine Flüssigkeit mit einer Oberflächenspannung von weniger als 21 mN/m benetzbar ist und die zweite Hauptoberfläche durch eine Flüssigkeit mit einer Oberflächenspannung zwischen 23 und 40 mN/m benetzbar ist.

4. Mikroporöse Membran nach einem der Ansprüche 1 bis 3, wobei der Hydrophobizitätsgradient durch die Anwesenheit einer fluorhaltigen organischen Verbindung erzeugt ist.

5. Mikroporöse Membran nach Anspruch 4, wobei die fluorhaltige organische Verbindung in Form eines an der mikroporösen Membran fixierten, Fluorsubstituenten enthaltenden Polymers vorliegt.

6. Mikroporöse Membran nach Anspruch 4, wobei die fluorhaltige Verbindung ein Reaktionsprodukt einer monomeren, oligomeren oder polymeren fluorhaltigen Verbindung mit reaktiven Gruppen des Ausgangsmaterials der Polymermembran ist.

7. Mikroporöse Membran nach einem der Ansprüche 1 bis 6, wobei die Polymermembran mindestens ein aromatisches Hauptkettenpolymer umfaßt.

8. Mikroporöse Membran nach Anspruch 7, wobei das aromatische Hauptkettenpolymer ausgewählt ist aus der Gruppe von PSU (Polysulfon), PES (Polyethersulfon) oder Gemischen hiervon.

9. Verwendung einer mikroporösen Membran nach einem der Ansprüche 1 bis 8 in der Sterilfiltration von gasförmigen Fluiden.

10. Verwendung einer mikroporösen Membran nach einem der Ansprüche 1 bis 8 als Flüssigkeitssperre in zu belüftenden flüssigkeitshaltigen Systemen.

## Claims

1. Microporous temperature-resistant polymer membrane, which resists gamma radiation, with two outer main surfaces, which are connected by the membrane body by way of micropores, and with a maximum temporary use temperature of at least 150° C, wherein the microporous membrane has a hydrophobicity gradient from the first outer main surface through the membrane body to the second outer main surface, wherein the hydrophobicity gradient is such that the hydrophobicity is at a maximum on the first outer main surface, initially decreases towards the second outer main surface, adopts a minimum within the microporous membrane body, but remains present, and subsequently increases again towards the second outer main surface without again attaining the maximum of the first outer main surface.

2. Microporous membrane according to claim 1, wherein the loss in strength of the polymer membrane due to γ radiation at a dose of 50 kGy is not more than 20%.

3. Microporous membrane according to one of claims 1 and 2, wherein the first main surface is wettable by a liquid with a surface tension of less than 21 mN/m and the second main surface is wettable by a liquid with a surface tension between 23 and 40 mN/m.

4. Microporous membrane according to any one of claims 1 to 3, wherein the hydrophobicity gradient is produced by the presence of an organic compound with a fluorine content.

5. Microporous membrane according to claim 4, wherein the organic compound with fluorine content is present in the form of a polymer fixed to the microporous membrane and containing fluorine substituents.

6. Microporous membrane according to claim 4, wherein the compound with the fluorine content is a reaction product of a monomer, oligomer or polymer compound, which has a fluorine content, with reactive groups of the starting material of the polymer membrane.

7. Microporous membrane according to any one of claims 1 to 6, wherein the polymer membrane comprises at least one aromatic main chain polymer.

8. Microporous membrane according to claim 7, wherein the aromatic main chain polymer is selected from the group of PSU (polysulfone), PES (polyethersulfone) and mixtures thereof.

9. Use of a microporous membrane according to any one of claims 1 to 8 in the sterile filtration of gaseous fluids.

10. Use of a microporous membrane according to any one of claims 1 to 8, as a liquid barrier in systems which contain liquid and are to be aerated.

## Revendications

1. Membrane polymère microporeuse résistant au rayonnement gamma, thermorésistante, comportant deux surfaces principales extérieures qui sont reliées par le corps de membrane par le biais de micropores, et ayant une température d'usage à court terme maximale d'au moins 150 °C, la membrane microporeuse présentant, de la première surface principale extérieure en passant par le corps de membrane à la deuxième surface principale extérieure, un gradient d'hydrophobicité qui est conçu de telle sorte que l'hydrophobicité est maximale sur la première surface principale extérieure, diminue d'abord jusqu'à la deuxième surface principale extérieure, se situe à un minimum à l'intérieur du corps de membrane microporeux, tout en étant encore présente, et ensuite augmente de nouveau jusqu'à la deuxième surface principale extérieure sans atteindre de nouveau le maximum de la première surface principale extérieure.

2. Membrane microporeuse selon la revendication 1, dans laquelle la perte de résistance de la membrane polymère par irradiation γ à une dose de 50 kGy n'étant pas supérieure à 20 %.

3. Membrane microporeuse selon l'une des revendications 1 ou 2, dans laquelle la première surface principale peut être humectée par un liquide ayant une tension superficielle inférieure à 21 mN/m et la deuxième surface principale peut être humectée par un liquide ayant une tension superficielle comprise entre 23 et 40 mN/m.

4. Membrane microporeuse selon l'une des revendications 1 à 3, dans laquelle le gradient d'hydrophobicité est généré par la présence d'un composé organique contenant du fluor.

5. Membrane microporeuse selon la revendication 4, dans laquelle le composé organique contenant du fluor se présente sous la forme d'un polymère contenant des substituants du fluor et fixé à la membrane microporeuse.

6. Membrane microporeuse selon la revendication 4, dans laquelle le composé organique contenant du fluor est un produit réactionnel d'un composé monomère, oligomère ou polymère contenant du fluor avec des groupes réactifs de la matière de départ de la membrane polymère.

7. Membrane microporeuse selon l'une des revendications 1 à 6, dans laquelle la membrane polymère comprend au moins un polymère aromatique de chaîne principale.

8. Membrane microporeuse selon la revendication 7, dans laquelle le polymère aromatique de chaîne principale est choisi dans le groupe de la PSU (polysulfone), PES (polyéther-sulfone) ou des mélanges de celles-ci.

9. Utilisation d'une membrane microporeuse selon l'une des revendications 1 à 8 dans la filtration stérile des fluides gazeux.

10. Utilisation d'une membrane microporeuse selon l'une des revendications 1 à 8 comme barrière de liquide dans des systèmes à ventiler contenant des liquides.
